# EUROPEAN PATENT SPECIFICATION

(11) **EP 1 474 245 B1**
(45) Date of publication and mention of the grant of the patent: **20.12.2006**
(21) Application number: 03707252.7
(22) Date of filing: 11.02.2003
(51) Int. Cl.: B05B 17/06

(54) **LIQUID SPRAY-HEAD, APPARATUS COMPRISING A LIQUID SPRAY-HEAD AND CONTAINER THEREFORE**
FLÜSSIGKEITSZERSTÄUBERKOPF, VORRICHTUNG MIT EINEM FLÜSSIGKEITSZERSTÄUBERKOPF UND ZUGEHÖRIGEM BEHÄLTER
TETE DE PULVERISATION DE LIQUIDE, APPAREIL POURVU D'UNE TETE DE PULVERISATION DE LIQUIDE ET RECEPTACLE CORRESPONDANT

(30) Priority: 11.02.2002 NL 0200053
(43) Date of publication of application: 10.11.2004
(73) Proprietor: Sara Lee/DE N.V., 3532 AA Utrecht (NL)
(72) Inventor: MIRO AMENOS, Jordi, E-08027 Barcelona (ES); MARTINEZ RODRIQUEZ, Elisabet, E-08010 Barcelona (ES)
(74) Representative: Winckels, Johannes Hubertus F.
(86) International application number: PCT/NL2003/000101
(87) International publication number: WO 2003/068413

(56) References cited:
- US-A- 4 605 167
- US-A- 4 702 418

## Description

The invention relates to a liquid spray-head according to the preamble of claim 1.

In US4702418 a liquid spray head is described with a piezo-element that sprays an aerosol from a membrane. To this, a diaphragm is provided with a nozzle centrally located. The piezo element is located on a wall opposite to the diaphragm and vibrates to eject fluid from the flexible membrane, in particular from the nozzle.

From EP-A-0615470 an apparatus is known for atomizing a liquid to be used, that is to say, for producing micro-droplets of liquid, for example, in an atomizing device for insecticide or air freshener liquid. The apparatus is based on a liquid ejecting membrane mechanically connected to an electromechanical actuator, preferably an electro-acoustic element, to make the membrane vibrate and means for supplying the liquid directly to a surface of the membrane. The liquid is atomized from the membrane due to its vibrating motion. The membrane can be perforated, in which case the liquid is atomized through it. Said means for supplying the liquid to the membrane comprise a capillary mechanism constituted by a wick made of open cell foam or fibre with a first end submerged in the liquid and a second end in contact with the membrane. The electro-acoustic actuator is formed by a laminar composite material comprised of a first active layer, which can be either an electrostrictive member, such as a piezoelectric, or a magnetostrictive member, and a second layer which can be either active or negative, mechanically joined. An enlarged field applied by means of electrodes makes that the first active layer tries to change its length in the direction of the plane, which causes a mechanic reaction with the second layer which provokes that the electro-acoustic actuator is bent. A driving circuit activates the electro-acoustic actuator in order it carries out a resonating vibration. According to an exemplary embodiment, the electro-acoustic actuator adopts the shape of an annular disk and the membrane is arranged at the central opening of annular disk. annular disk and the membrane is arranged at the central opening of annular disk.

From US patent 3.790079 a liquid spraying head is known having a fixed connection between the liquid providing member and the liquid ejecting member. A supply tube delivering a sprayable liquid is directly coupled to a membrane. Such a fixed delivery arrangement has as a disadvantage that the piezo-element is not freely suspended by it hindered by the supply tube. Furthermore, the liquid ejecting member only vibrates in bending modes, since the perimeter is fixed to the housing by a fixing ring.

The object of this invention is to supply an liquid spray-head which provides a state of contact without tightening or load between the liquid providing member and the liquid ejecting member in order that a contact under an excessive or incorrect pressure of the wick on the liquid ejecting member does not interfere in the vibrating motion of said liquid ejecting member at same time that the supply to the liquid ejecting member is carried out in an even and regular way, all of it regardless the service conditions or service slope positions of the device.

The above mentioned object is achieved by a liquid spray-head according to claim 1.

With this arrangement, the flexible member supports the unit of the vibrating actuator and the liquid ejecting member so that the top end of the liquid providing member, and a contact side of the liquid ejecting, member is kept in a contact state without tightness nor load which prevents that a contact under an excessive or incorrect pressure of the capillary sucking member on the liquid ejecting member interferes in the vibrating motion of the liquid ejecting member and makes that the supply of the liquid to the liquid ejecting member is even and continuous, regardless of the service conditions or service slope of the device. In addition, the arrangement of the flexible member provide a longer useful life to the liquid ejecting member and a greater flexibility which allows to place the device in different conditions or service slope positions.

In one embodiment, the flexible member is annulus-shaped, extending around the liquid ejecting member. The vibrating actuator may have a conventional disk or ring form enclosing or at least partially covering the liquid ejecting member. This embodiment provides the advantage that a uniform flexibility around the liquid ejecting member is provided. In this way, the liquid ejecting member is freely suspended, so that the liquid ejecting member can vibrate freely against the liquid providing member. An adequate flexibility is further provided by the flexible member being provided with a radial cross-section showing at least one undulation between a central opening and its periphery.

Preferably, the flexible member is provided with a radial cross-section extending from an outer perimeter of said liquid ejecting member to an inner wall of the housing for providing a flexible connection between said housing and said liquid ejecting member. Such a flexible connection allows a certain amplitude of lateral movement along a line extending from the perimeter of the liquid ejecting member to the inner wall of said housing so as to freely suspend the liquid ejecting member.

Preferably the flexible member is cone-shaped, the cone extending in a direction normal to an ejection side of the liquid ejecting member. The cone shape provides an improved working contact between the liquid ejecting member and the liquid providing member. Additionally, the cone may provide a directing effect for directing the spray in a substantial normal direction to the ejection side of the liquid ejecting member.

In a further embodiment, the flexible member comprises inwardly extending surface areas contacting opposite surfaces of said liquid ejecting member so as to enclose said liquid ejecting member. In this way, by providing a cost effective way to secure the liquid ejecting member to said flexible member the number of constituting parts of the spray-head can be minimized. As an alternative, the flexible member is provided with a inner perimeter that is matingly connected to a rim of the liquid ejecting member. As a further alternative, the flexible member is provided with an inner perimeter comprising a plurality of through-holes, the spray-head further comprising an enclosing member comprising protrusions entering said through-holes, for enclosing said liquid ejecting member by said enclosing member and said flexible member. Further, the inner perimeter comprises a notch for passage of connecting wires of the vibrating actuator.

In yet another embodiment, the flexible member is provided with a perimeter that is sealingly connectable to a rim of an opening of a liquid containing container. In this way, the flexible member seals off an opening of the container, thereby minimizing leakage or spill. To provide a better sealing effect, the flexible member is provided with a reinforced perimeter.

In a further embodiment, the spray-head is provided with a liquid providing member, the liquid spray-head further comprising an annular fixing member, for fixing said liquid providing member relative to the liquid ejecting member. This embodiment provides a stable and uniform positioning of the liquid providing member in relation to the flexible member and the liquid ejecting member contained thereby. For better liquid providing properties, said liquid providing member has a chamfered surface to be contacted with the liquid ejecting member. The chamfered surface the surface provides a better and more durable flow of liquid to the perforations of the liquid ejecting member.

Advantageously, the flexible member is moulded from an elastomeric material from a group comprising silicones, fluorinated elastomers, fluorosilicones, nitrile-butadiene (NBR), hydrogenated nitrile-butadiene (HNBR), thermoplastic polyester-elastomer, neoprene (cloroprene), terpolimer of etylpropilene (EPDM) and EPDM peroxide. Preferably, the elastomeric material is the fluorinate elastomer FKM 60. In yet another preferable embodiment, the flexible member is monolithically formed to the housing, thus providing a cost effective way of producing said spray-head.

The invention also relates to container for comprising liquid, to be secured to a liquid spray-head according any of the above describe features. In particular, the invention relates to a container comprising a liquid providing member and an annular fixing member, for fixing said liquid providing member. Preferably, the fixing member comprises an outer surface providing a sealing contact between the periphery of a flexible member of a spray-head and an inner surface fixedly contacting said liquid providing member. The liquid providing member preferably comprises a chamfered surface to be contacted with the liquid ejecting member.

In another respect, the invention relates to an apparatus for spraying a liquid, comprising a liquid spray-head according to any of the above aspects. Such an apparatus may comprise an electrical control circuit for controlling the liquid spray head, the control circuit comprising a timer circuit and/or an intensity switch. Furthermore, the apparatus may comprise a fan and a conduit for ducting fanned air over the spray-head. In a preferred embodiment, the apparatus is arranged to deliver fragrances. In this way, the apparatus can be used as an air freshener. Especially since no heating is involved in transporting the fragrances into the atmosphere, more sophisticated fragrances can be used that will not suffer from degradation by heat. Furthermore, the delivery system using the liquid spray head of the above described aspects offers an effective dose control that can be adjusted to certain timing (for instance: delivering a fragrance during a predetermined interval) and intensity.

In another embodiment the apparatus according the invention is an apparatus for exterminating crawling insects, comprising a liquid spray-head according to any of the preceding claims, a fan, an electrical control circuit and a conduit for ducting fanned air over the spray-head so as to provide a directed spray.

A preferred embodiment of said apparatus is provided by the fan comprising a rotor wheel having an axis of rotation parallel to an ejecting direction of the spray-head, wherein the conduit deflects fanned air in a direction transverse to said ejection direction. This embodiment allows for an especially flat design of the apparatus, allowing for placement in spaces having low altitude, like for instance under a cupboard etc.

The control circuit may be arranged to control the spray-head in at least two different operation modes previously programmed and stored in a memory associated to said control circuit, wherein in a first operation mode a first predetermined dose of liquid is sprayed during a first predetermined period of time for detection of crawling insects, and wherein in a second operation mode a second predetermined dose of liquid is sprayed during a second predetermined period of time, separated by a predetermined period from said first predetermined period of time, for effective extermination of crawling insects. Said operation modes may be selectable by a selector switch.

Further advantages and features will become apparent when reading the description in connection with the drawings. In the drawings:
Fig. 1 shows a perspective view of a preferred embodiment of an apparatus comprising a liquid spray-head according to the invention;
Fig. 2 is a diametral cross sectional view of the mounting of the liquid spray-head of this invention using a diaphragm according to a first exemplary embodiment;
Fig. 3 is a plan view of the diaphragm of Fig. 2;
Fig. 4 is a diametral cross section of the mounting of the liquid spray-head of this invention using a diaphragm according to a second exemplary embodiment;
Fig. 5 is an exploded perspective view of part of the mounting of Fig. 4.
Fig. 6 contains a diagram of a diametrical section of the piezoelectric device mounting, made up of a central frame, an annular membrane and a perimeter frame.
Fig. 7 represents one half of a diametrical section of a mounting for the piezoelectric device in which the membrane is made up of ridges and troughs of the zigzag type.
Fig. 8 represents a similar view to Fig. 7 with the membrane having wider, less frequently occurring ridges and troughs.
Fig. 9 represents a similar view to Fig. 7 and 8, in which the membrane has narrower, more frequently occurring ridges and troughs.
Fig. 10 represents a similar view to Figs. 7-9, in which the membrane has ridges and troughs of the undulated type.
Fig. 11 represents a similar view to Figs 7-10, in which the annular membrane is represented by its upper surface, in one case, by a continuous dotted line forming a single ridge with a curvilinear arched straight cross section and, in another case, by a broken dotted line forming a single trough having a curvilinear arched straight cross section.
Fig. 12 represents a similar view to Figs. 7-11 considered above, in which the annual membrane has, on its upper surface an exterior ridge and an inner trough, both concentrically shaped and of the undulating type.
Fig. 13 represents diagramatically an assembly of a device provided with a liquid container, the device incorporating a spray-head of the invention and forming a support enabling the workable positioning of a container holding the spray liquid;
Fig. 14 shows a plane view and side views of a preferred connection between the liquid ejecting member and the flexible member;
Fig. 15 shows a side view of a preferred embodiment of the flexible member and of the liquid providing member;
Fig. 16 is a view in perspective of the apparatus for controlling a crawling insect population of this invention;
Fig. 17 is a top plane view of the apparatus of Fig. 18 with the cover withdrawn to show its interior;
Fig. 18 is a cross sectional view, with the cover included, taken across the plane III-III of Fig. 17 in the direction of the arrows;
Fig. 19 is a diagram of the control electronic circuit of the apparatus operation; and
Fig. 20 and 21 illustrate the method of the invention by means of schematic diagrams corresponding to the spreading flow rate of the active substance depending on the time in two different operation modes of the apparatus.

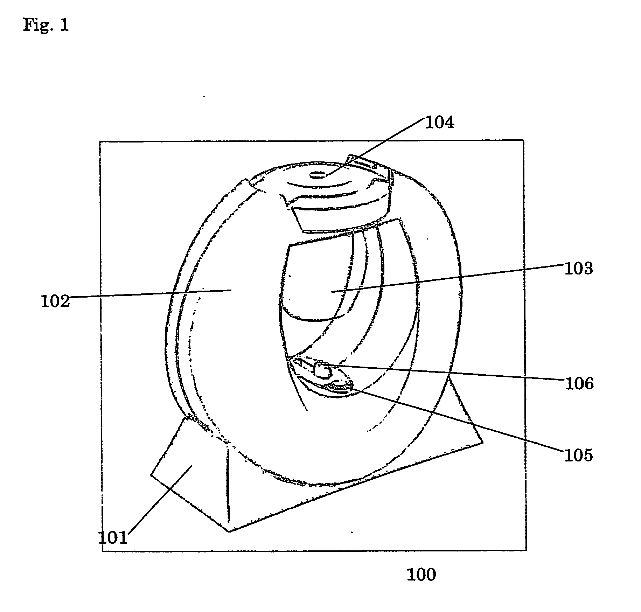

In figure 1, a perspective view is shown of an apparatus comprising a liquid spray-head according to the invention. The apparatus in fig. 1 is designed as a table piece 100, comprising a base plate 101 and a structure 102 for holding a liquid container 103. The liquid container 103 contains a liquid that produces an odour that can for instance be used for freshening the atmosphere. The liquid container 103 may be a refillable container or may be a replaceable unit that can be inserted once the container is empty. The liquid spray head will be further illustrated in subsequent figures; in figure 1 an opening 104 is shown wherefrom liquid spray is ejected by the liquid ejecting head. The apparatus 100 is further provided with an on/off button 105 and a sliding contact 106 for controlling the intensity of the ejected spray. The preferred embodiment is provided with a timing circuit, that automatically turns the liquid spray off after a predetermined time, to prevent excessive spraying of fragrance.

Where conventional fresheners usually utilize heating for vapourizing liquids and for transporting the fragrances into the atmosphere, the apparatus according to the invention offers the advantage that no heating is involved. Hence, more sophisticated fragrances can be used that will not suffer from degradation by heat. Furthermore, the vaporizing action of the liquid spray-head according to the invention is immediate, hence an almost immediate effect is sensed when the apparatus according to the embodiment of fig. 1 is turned on.

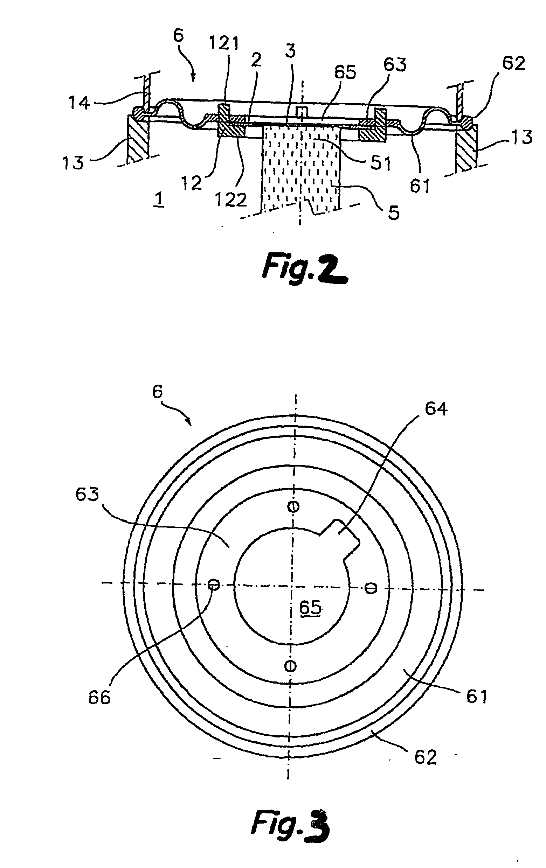

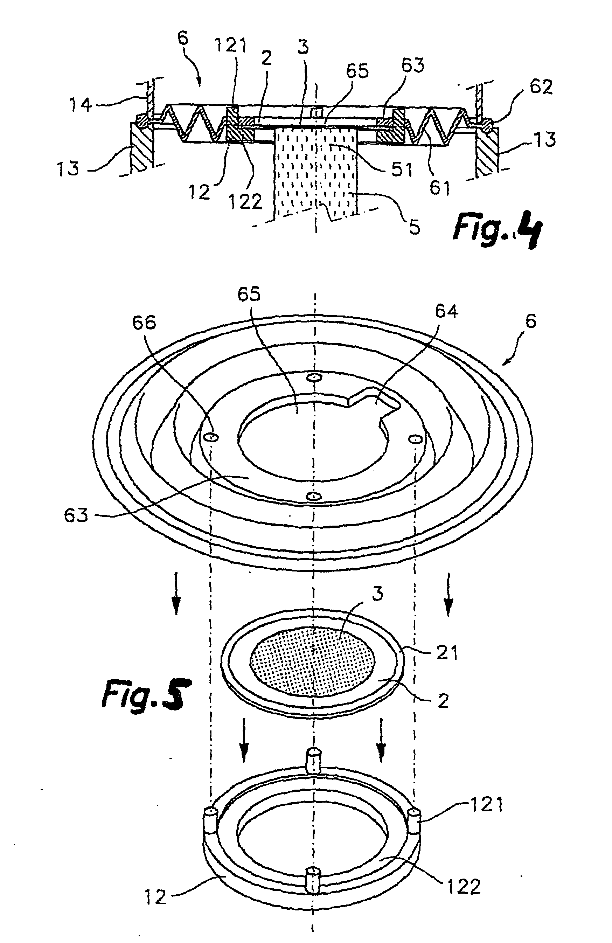

In fig. 2, the atomizing ultrasound spray-head comprises: a housing 13, to be secured to a container containing a sprayable liquid; a circular perforated liquid ejecting membrane 3 connected to said housing 13, to be contacted with a liquid providing wick 5 for providing liquid to membrane 3; and a ultrasound vibrating annular actuator 2 connected to said membrane 3 for actuating said membrane 3 so as to generate a spray of liquid. The ultrasound vibrating annular actuator 2 is composed of an active layer and a reaction layer, the circular perforated membrane 3 is coupled to a central opening of said vibrating annular actuator 2 to be vibrated by it and the capillary liquid providing member or wick 5 for supplying by capillarity said liquid directly to a surface of said perforated membrane 3. Typically, the wick 5 is of open cell foamy polymeric material or of fibrous material and has a lower end submerged within a liquid contained in a container (not shown) and a top end 51 which is the one that contacts membrane 3. The liquid is atomized through the perforated membrane 3 by the effect of the vibration thereof produced by the vibrating annular actuator 2. According to a preferred application, not shown, a conveniently oriented airflow assists to spreading the atomized liquid.

As it is shown in Fig. 2, the spray-head comprises a flexible diaphragm 6 extending from an outer perimeter of said membrane 3 to an inner wall of the housing 13 for providing a flexible connection between said housing 13 and said membrane 3. In this way, as is apparent from the figure, such flexible connection allows a certain amplitude of lateral movement along a line extending from the perimeter of the membrane 3 to the inner wall of the housing. Thus, by such a flexible connection, the liquid ejecting member is freely suspended, so that the liquid ejecting member can vibrate freely against the liquid providing member. The vibrating annular actuator 2 bearing the perforated membrane 3 is fastened on a central opening 65 of an annulus shaped elastic diaphragm 6 extending around the membrane 3, which is supported on a housing 13, 14 or the like. Said container containing the liquid, to which the wick 5 is associated is also linked to said housing 13, 14. In the example illustrated, a thickening 62 of the periphery of the diaphragm 6 is seated in a recess of a lower support 13 of said housing and a upper support 14, which is integral with, for example a cover of the housing, tighten said thickening 62 or an adjacent area of the diaphragm 6 against the lower support 13.

Thus, the diaphragm 6 supports, thanks to its elasticity, the unit of the vibrating annular actuator 2 and the perforated membrane 3 so that the wick 5 and the surface of the membrane 3 are kept in a state of contact without tightening nor load so that the wick 5 does not interfere in the vibrating motion of the membrane 3 and that the supply by capillarity of the liquid to the membrane 3 is even and continuous, regardless the service conditions or service slope positions of the device.

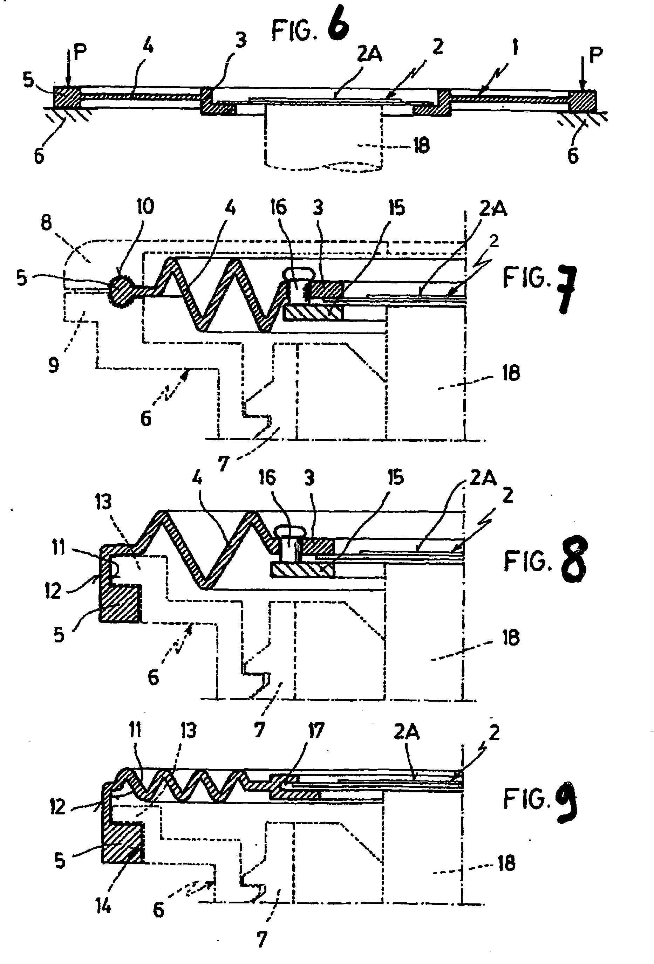

The elasticity of the diaphragm 6 is provided by a combination of material and geometric shape. Thus, on one hand, the diaphragm 6 is of an elastomeric material selected from a group comprising silicones, fluorinated elastomers, fluorosilicones, nitrile-butadiene (NBR), hydrogenated nitrile-butadiene (HNBR), thermoplastic polyester-elastomer, neoprene (cloroprene), terpolimer of etylpropilene (EPDM) and EPDM peroxide, preferably the fluorinate elastomer FKM 60, and on the other hand, the diaphragm 6 is annulus-shaped (see Fig. 3 and 4) and is provided with a radial cross section showing one or more undulations 61 between said central opening and its periphery which provides them an elasticity very sensible to the vibrating motions which extends the useful life of the membrane and facilitates placing the device in different service slope positions. According to an exemplary embodiment shown in Fig. 2 and 3, and as it can be seen in said radial cross section shown in Fig. 2, said undulation 61 has a zigzag profile with rounded sides and comprises a single cycle. In Fig. 4 and 5, another embodiment is shown in which the profile of the cross section of the diaphragm 6 has straight sides and rounded vortices and comprises two complete cycles. Other configurations of the profile and/or other number of cycles are possible as is apparent from embodiments shown by way of example in figs. 6-15.

In both cases, the undulated area of said diaphragm 6 has a constant thickness while on the periphery it shows said reinforcing thickening 62 and on an area close to the central opening 65, a fastening configuration 63, 66 for mounting the vibrating annular actuator 2. Optionally, the diaphragm 6 in addition includes a notch 64 (see Fig. 3 and 5) on the edge of the central opening 65 for the passage of connecting wires of the vibrating annular actuator.

Fig. 5 shows the characteristics of said mounting. In said fastening configuration a flat portion 63 is included around said central opening 65 where through holes 66 are provided in which stubs 121 arranged on the periphery of a mounting ring 12 are pressure inserted. Said mounting ring 12 is provided with a recessed flat portion 122 located around a central opening, a metallic ring 21 for supporting the peripheral edge of the vibrating annular actuator 2 remains trapped between both flat portions 63, 122 of the diaphragm 6 fastening configuration and the mounting ring 12.

Fig 6 shows a diaphragm 1 for a electro-acoustic type piezoelectric device 2, which is made up of a central frame 3, a resilient sheet 4 and a perimeter frame 5. The diaphragm 1, central frame 3 , resilient sheet 4 and perimeter frame 5 form an integral unit obtained from synthetic elastomeric resin, preferably fluor elastomer, such as "VITON" by Du Pont, but this does not exclude the possibility of using, albeit with varying results, silicones, polyurethane elastomer, fluorosilicones, NBR (Nitrile Butadiene Rubber), thermoplastic polyester-elastomer, HNBR (Hydrogenate Nitrile Butadiene Rubber), neoprene (chloroprene), EPDM (Ethylene-Propylene Terpolymer rubber) and EPDM peroxide.

Diaphragm 1 is fitted by any conventional means through pressure P that is applied to the whole surface of perimeter frame 5 in order to apply it to a housing 6 which, forming part of a spray apparatus that is not represented herein, enables a container 7 holding the spray liquid to be positioned in a workable manner by means of a thread engagement, bayonet engagement etc.

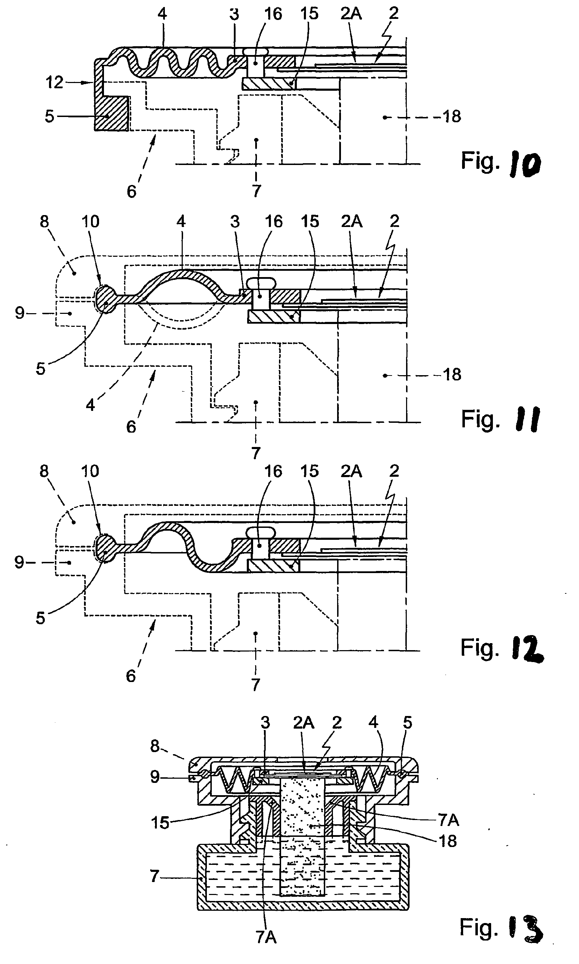

In Fig 7, diaphragm 1 is fixed to housing 6 by means of a clamping device consisting of two jaws 8 and 9 which fix bead 10 in which perimeter frame 5 is formed.

In Figs 8, 9 and 10, this fixing arrangement is achieved by a flexible diaphragm provided with a skirting 12 having a groove 11 that is sealingly connectable by snap engagement to a projection 13 or notch 14 of an opening of a liquid containing container.

Piezoelectric device 2 can be fixed to central frame 3 by means of an adhesive, as in Fig 6, by means of a central ring 15 that is joined to central frame 3 by welding, or by means of riveted lugs 16, or the like, that firmly fix the piezoelectric device between them, as in Figs 7, 8, 10-12 or, by means of an annular housing 17 which, like a pocket, is formed within central frame 3 proper, or incorporated therein. In this way the diaphragm 1 is provided with an inner perimeter comprising a plurality of through-holes, so that an enclosing annular housing 17 comprising lugs 16 enters said through-holes, for enclosing the piezo-electric device holding nozzle 2A by said enclosing member 17 and said diaphragm 1.

As shown in Figs 6-12, by means of mounting 1 for piezoelectric device 2, it is possible that nozzle 2A is always applied to the head of a liquid providing wick 18, by maintaining a thin layer of spray liquid always in contact with nozzle 2A of piezoelectric device 2, which does not need any priming. At the same time, owing to the gentle, controlled pressure with which said nozzle 2A is applied to the head of wick 18 provided by the flexibility and elasticity of mounting 1, determines the correct operation of the spray function.

In Fig. 13 a spray-head is illustrated having a liquid container 7. In this arrangement, the container 7 comprises a liquid providing wick 18, and an annular fixing member 7A, for fixing said wick relative to the nozzle 2A. Hence the fixing member 7A forms a support enabling the workable positioning of wick 18. As will be understood, the invention is also related to charactering features of the container per se.

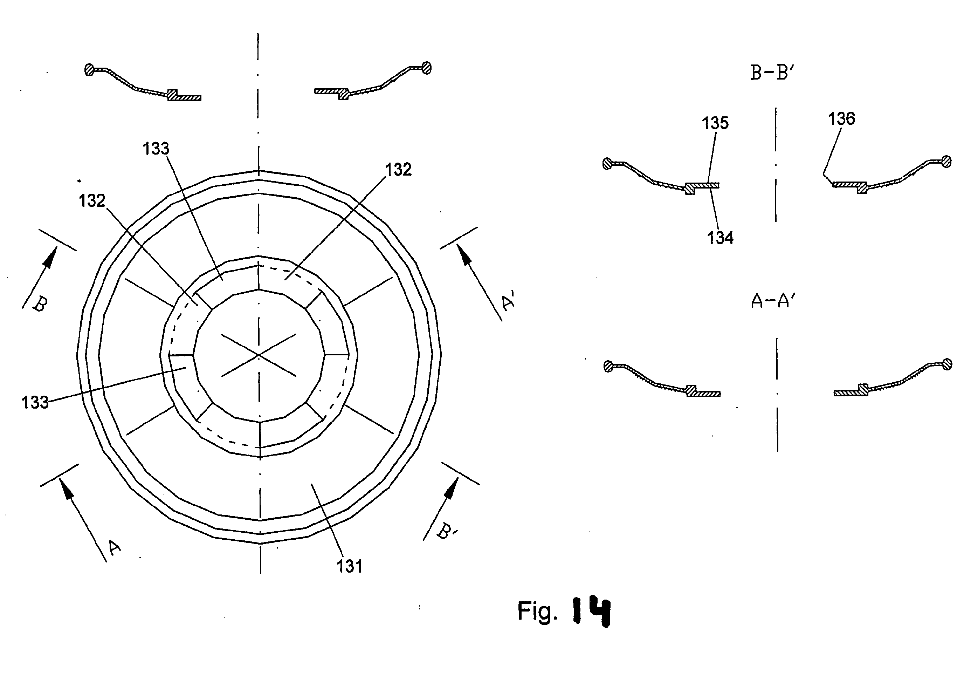

Fig. 14 shows a plane view and side views of an embodiment wherein the diaphragm 131 comprises inwardly extending surface areas 132, 133 contacting opposite surfaces 134, 135 of said liquid ejecting member 136 so as to enclose said liquid ejecting member 136.

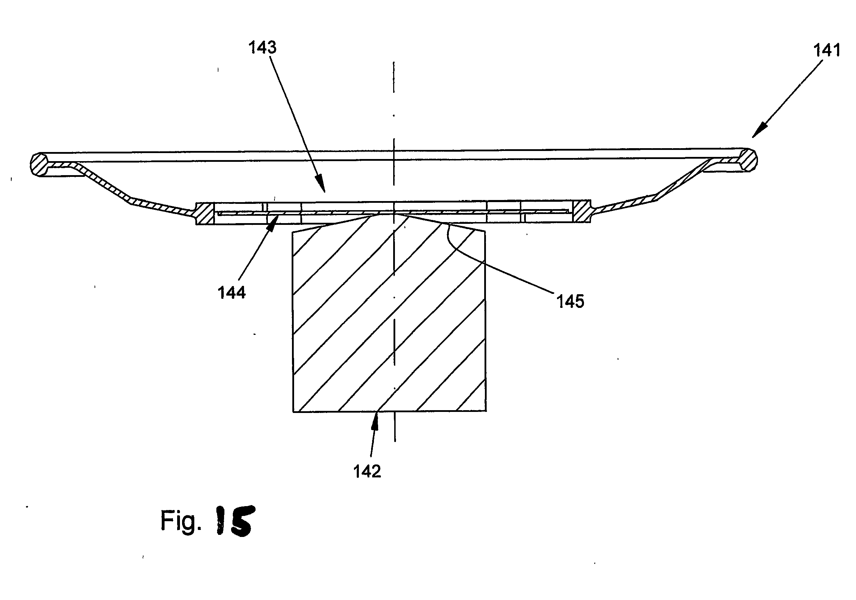

Fig. 15 shows a side view of a preferred embodiment of the flexible member 141 and of the liquid providing member 142. The flexible member is cone-shaped, the cone extending in a direction normal to an ejection side 143 of the liquid ejecting membrane 144. The cone shape provides an improved working contact between the membrane 144 and the wick 142. Additionally, the cone may provide a directing effect for directing the spray in a substantial normal direction to the ejection side 143 of the membrane 144. The liquid providing member or wick 142 shown in fig. 15 comprises a chamfered contacting surface 145 for contacting the liquid ejecting member 144. The chamfered surface 145 provides a better and more durable flow of liquid to the liquid ejecting member 144.

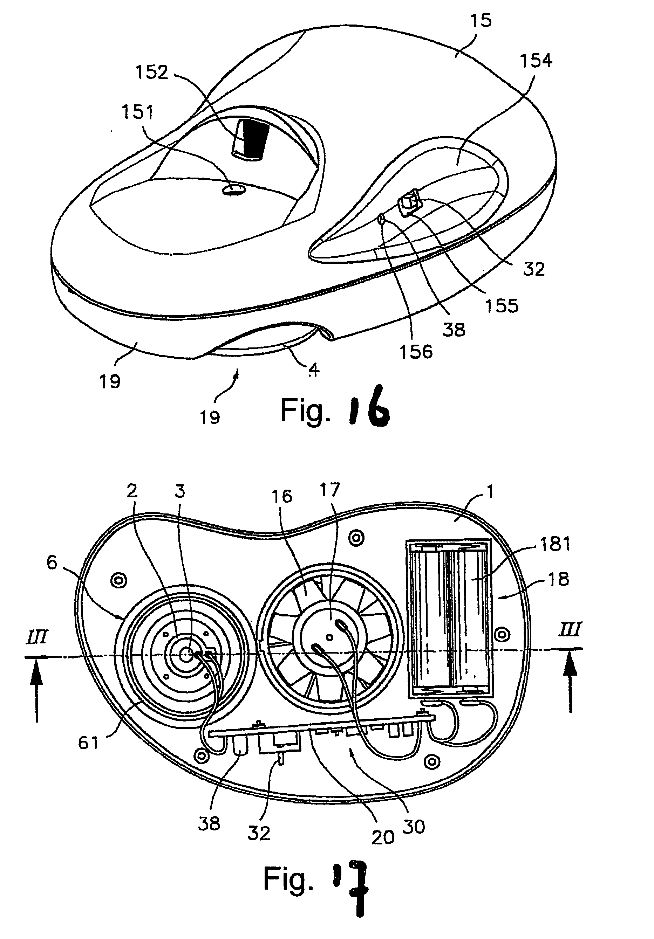

Referring to Figs. 16 to 18, in which a preferred embodiment of the apparatus for exterminating crawling insects according to the invention is shown, comprising a liquid spray-head 2, a fan 16, an electrical control circuit 20 and a conduit 153 for ducting fanned air over the spray-head 2 so as to provide a directed spray. The housing of the apparatus is embodied by a bottom 1 and a cover 15 which jointly form an internal enclosure serving as support and protection to the remaining components forming the apparatus.

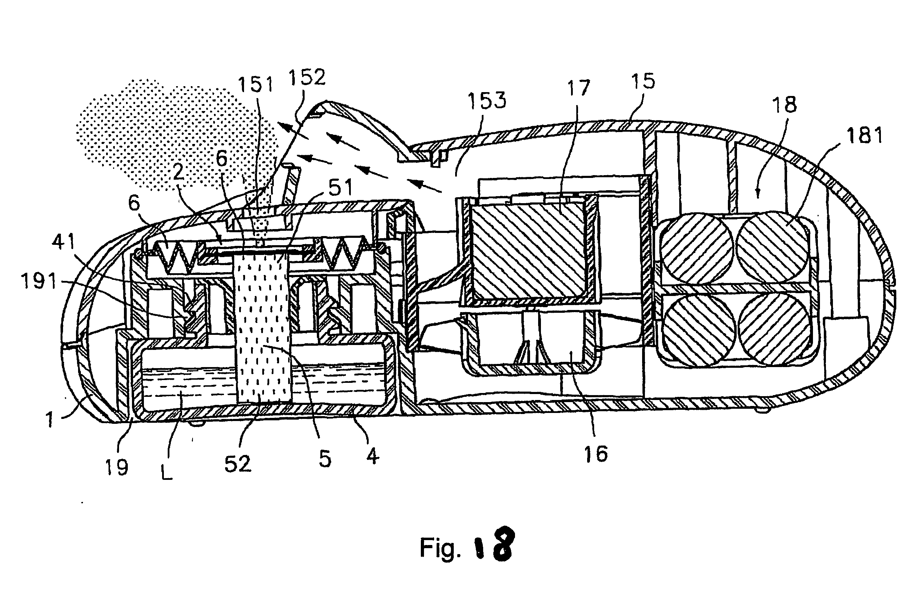

Said components contained in the housing 1, 15, basically include a piezoelectric atomizer 2, a container 4 for a liquid active substance L; a capillary sucking member, such as a wick 5, for providing this liquid active substance L from said container 4 to the piezoelectric atomizer 2; a fan 16; electronic means 30 for controlling the operation of the piezoelectric atomizer 2 and fan 16; and a power supply 18 for supplying the different components which require it. In the embodiment illustrated in figs. 17 and 18, the fan 16 comprises a rotorwheel having an axis of rotation parallel to an ejecting direction of the spray-head 2, wherein the conduit 153 deflects fanned air in a direction transverse to said ejection direction. This embodiment allows for an especially flat design of the apparatus, allowing for placement in spaces having low altitude, like for instance under a cupboard etc.

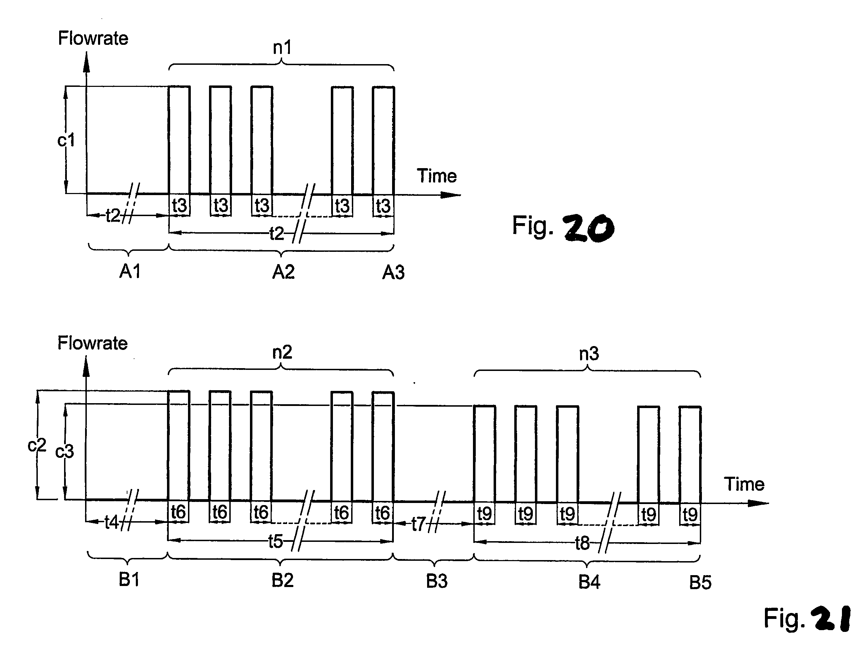

Said control electronic means 30 are advantageously incorporated in a printed circuit board 20 which in addition incorporates a selector switch 32 and a power on light 38. This selector switch 32, which is accessible from outside the housing 1, 15, allows that the user starts or stops the apparatus and selects a specific operation mode from at least two different operation modes previously programmed and stored in a memory associated to the control electronic means 30. These operation modes are adapted for carrying out different tasks required for an effective extermination of the crawling insects and will be described below with respect to Fig. 20 and 21.

The piezoelectric atomizer 2 comprises an ultrasonic vibrating annular actuator, composed of a active layer and a reaction layer and a circular multiperforated membrane 3 connected to a central opening of that vibrating annular actuator to be vibrated by it. The piezoelectric atomizer 2 is supported by new elastic or flexible means, provided by a diaphragm 6, which makes sure a contact without tightness or load of the wick 5 and the surface of membrane 3. This makes that the wick 5 does not interfere in the vibrating motion of the membrane 3 and that the supply by capillarity of the liquid to the membrane 3 is even and continuous, regardless the conditions or positions of the operating slope of the device. The elasticity of the diaphragm 6 is provided by a combination of the elastomeric material of which it is made and its circular crown geometric shape which includes one or more circumferential concentric waves 61 between a central opening 65 and its external periphery 62.

Said container 4 containing the liquid L incorporates a wick 5 and the assembly is housed in a recess 19 provided for that purpose at the lower part of the housing 1, 15, in such a position that a top end 51 of the wick 5 is contacting the membrane 3 while a lower end 52 of it is submerged in the liquid L. Said recess 19 is accessible from outside, as shown in Fig. 16 so that the assembly of container 4 and wick 5 is easily replaceable. Fastening the container 4 to the bottom 1 of the housing is carried out by respective threadings 41, 191.

The cover 15 shows a first opening 151 located on the multiperforated membrane 3 of the piezoelectric atomizer 2, for allowing the jet of atomized L active substance go out through it and a second opening communicated with an internal space 153 of the housing where said fan 16 is housed, for allow an air flow (shown by arrows) go out and laterally hit said jet of atomized L substance just above the first opening 151 in order to spread the atomized L substance in a limited extension area adjacent to the spreading apparatus.

The cover 15 in addition comprises a depression 154 on one side, in that depression a first window 155 is arranged through which there is an access to the selector switch 32 and a second window 156 through which said power on light 38 is visible, said selector switch 32 and power on light 38 being incorporated together with the electronic means 30 on the printed circuit board 20 housed and fastened within the housing 1, 15 in a suitable position, as it has been described above.

Preferably, said power supply 18 comprises one or several concatenated batteries 181 housed in that housing 1, 15, which provides the apparatus with a very advantageous autonomy. However, the power supply could also comprise, for example, a transformer for adapting a current coming from outside through a connecting cable (not shown).

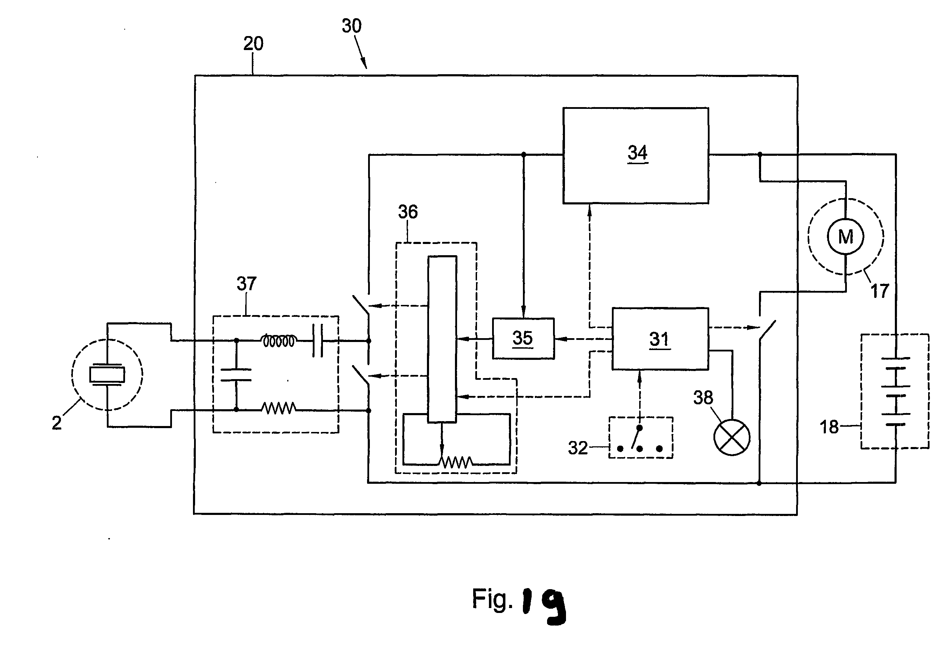

In Fig. 19 the electric circuit of the apparatus of this invention is shown including control electronic means 30 incorporated in said printed circuit plate 20.

The components incorporated on the printed circuit board 20 include a programmable integrated circuit (PIC) 31 which allows to activate and deactivate subsystems required according to one of the different operation cycles programmed and designed for correctly supplying active substance according to said different programs. Said selector switch 32 and the power on light 38, which is in the form of an emitting light diode (LED), are connected to that programmable integrated circuit 31. Connected to said batteries 181 a voltage booster regulator (VBR) 34 is arranged which can be an amplifier or transformer, the function of which is to boost the voltage of concatenated batteries 18 to the voltage necessary for supplying an oscillator circuit mentioned below. Said voltage booster regulator 34 is controlled by PIC 31. A linear regulator 35, supplied by the line at increased voltage and regulated by said VBR 34, has the function to transform this voltage into the voltage required for a subsequent oscillator circuit (RCL) 37. The linear regulator 35 is also controlled by PIC 31. Said oscillator circuit 37 is controlled by an oscillator control device 36 the function of which is to commute the oscillator circuit to the mains or towards the earth. It is controlled by PIC 31 and it is supplied by the voltage generated by the linear regulator 35. This device in addition possesses a potentiometer allowing to set the final resonance frequency within a pre-established range. Last, the oscillator circuit 37 is composed of resistors, coils and condensers, which generate an oscillation at a given frequency of resonance. Its on/off state is controlled by said oscillator control device 36 for generating excitation pulses of the mentioned piezoelectric atomizer 2. Motor 17 driving the fan 16 is supplied at the voltage provided by the batteries 18 and its operation is also controlled by PIC 31.

The procedure for controlling a crawling insect population according to this invention is described below with reference to Fig. 20 and 21.

The procedure essentially comprises spreading at least one active substance, specific for said insects, by means of an electronically controlled spreading apparatus, such as the apparatus of this invention described above, for dosing said substance in an area having a limited extent where it is assumed or sure that there exists a population of said crawling insects, such as cockroaches. The control circuit 30 is arranged to control the spray-head 2 in at least two different operation modes previously programmed and stored in a memory associated to said control circuit, wherein in a first operation mode a first predetermined dose of liquid is sprayed during a first predetermined period of time for detection of crawling insects, and wherein in a second operation mode a second predetermined dose of liquid is sprayed during a second predetermined period of time, separated by a predetermined period from said first predetermined period of time, for effective extermination of crawling insects.

Said operation modes include a detection mode, to be applied in an area where it is assumed that there exists a population of said crawling insects for confirming the existence or no existence of said population in said area, and a treatment mode, to be applied in an area where it is sure there exists a population of said crawling insects, for exterminating or controlling said population in that area.

These two operation modes of the apparatus allow to apply the full procedure of the invention including the detection and further treatment of the insect plague. The procedure comprises, to start initially the spreading apparatus with said detection mode selected and thereafter place the spreading apparatus prepared this way in a first area where it is assumed there exists a population of said crawling insects. If in said first area submitted to the detection mode there appears a given number of crawling insects, then start the spreading apparatus with said selected treatment mode and thereafter place again the spreading apparatus thus prepared in that first area where the existence of the crawling insect population has been detected. If, on the contrary, there appears no insects in the first area, then start again the spreading apparatus with said selected detection mode and thereafter place the spreading apparatus thus prepared in a second area where it is also assumed that there exists a crawling insect population and so on until finishing the inspection and treatment in the suspected areas.

Fig. 20 shows a diagram of flow rate against time corresponding to operation of the spreading apparatus in the detection mode, which, once started follows steps A1) to A3) described hereinafter. A first step A1) consists in remaining idle for a predetermined period of time 11, considered sufficient for allowing an user to place the apparatus in the selected area without being affected by a premature spreading of said active substance. Following step A2) consists in spreading the active substance during a period of time 12 by means of a pulse sequence n1 of predetermined flow rate c1, frequency and time t3 until completing a dose considered sufficient for having the crawling insects going out of their hiding places. Finally, step A3) consists in stopping the operation of the spreading apparatus, with which the inspection step is finished and the user can withdraw the apparatus from the inspected area and proceed to inspecting a new area or proceed to treating that area.

In Fig. 21 a diagram of flow rate against time is shown corresponding to the operation of the spreading apparatus in the treatment mode, which, once started, follows steps B1) to B5) described hereinafter. A first step B1) comprises remaining idle for a predetermined period of time 14, considered sufficient for allowing an user to place the apparatus in the selected area without being affected by a premature spreading of said active substance. Following step B2) consists in spreading the active substance during a time t5 by means of a pulse sequence n2 of predetermined flow rate c2, frequency and time t6, until completing a dose considered sufficient for effecting a lethal shock on the crawling insects. Then, a step B3) comprises remaining idle again during a second period of time t7 considered sufficient to allow any egg or latent larva is metamorphosed into an insect. Following step B4) consists in subsequently spreading the active substance during a predetermined period of time t8 by means of a sequence of predetermined flow rate c3 pulses, frequency and time t9 until completing a subsequent dose considered sufficient for producing a lethal effect on the surviving insects of the prior atomizing period of time or metamorphosed thereafter. Finally, the last step B5) consists in stopping the operation of the spreading apparatus, after which the treatment is finished and the user can withdraw the apparatus from the area treated.

Advantageously, the treatment mode includes repeating steps B3) and B4) a given number of times considered sufficient for maintaining said lethal effect before proceeding to the last step B5).

Although the active substance can be of a single component, it is advantageously formed from a mixture of different components leading to achieve different effects on the crawling insects. Preferably, the active substance used with the procedure and the apparatus of this invention is a mixture of at least three components at least one of them selected from the group of the pyrethroids type. Each of the three components is engaged in achieving one of the following effects on the crawling insects: hyperactivity; elimination; and disruption of the reproduction cycle. The three components are in said mixture in suitable proportions in order that each is effective when the mixture is spread at one of the different conditions of programmed dosing. That is to say, the first component is active for affecting the insects when the mixture is dosed in accordance with the detection mode, the second component is effective for exterminating the insects when the mixture is dosed in accordance with step B2) of the treatment mode and the third component is effective for disrupting the reproduction cycle of the insects when the mixture is dosed in accordance with steps B2 and B4) of the treatment mode.

By way of example, and only for offering an order of magnitude of the parameters under which the apparatus acts in accordance with the different operation modes, indicative values are provided for that parameters.

In the inspection mode (Fig. 20), the initial waiting time t1 can be approximately 2 minutes, the pulses sequence n1 can include approximately 17 pulses with a time t3 of 5 s at a frequency of 10 s and releasing a flow c1 of approximately 3 mg of active substance per second, which means applying a dose of approximately 255 mg along a time t2 of 165 seconds.

In steps B1) and B2) of the treatment mode (fig. 21), the initial waiting time t3 can be approximately 4 hours, the pulses sequence n2 can include approximately 228 pulses with a time t6 of approximately 5 s at a frequency of 10 s and releasing a flow of approximately 3 mg of active substance per second, which means applying a dose of approximately 3420 mg along during a time t5 of 37 minutes and 55 seconds.

Last, in steps B3) and B4) of the treatment mode (Fig. 21), the off time t7 can be approximately 7 days, the pulses sequence n3 can include approximately 114 pulses with a time t9 of approximately 5 s at a frequency of 10 s and releasing a flow of approximately 3 mg of active substance per second, which means applying a dose of approximately 3420 mg during a time t8 of 37 minutes and 55 seconds.

However, it must be stated that other dosing parameters are possible, as well as other variations in the procedure and apparatus. Although the invention has been illustrated with reference to piezo-electric actuators, other type of actuators, such as electro- or magnetostrictive actuators may be used. Further, various variations may be possible, for instance, a separate fixing ring placed in the container for fixing the liquid providing member, a ring placed between the container opening and the flexible diaphragm, a connecting element connecting the diaphragm and the liquid ejecting member. These and other variations are deemed to fall in the scope of the invention, as defined in the annexed claims.

## Claims

1. Liquid spray-head, comprising:
- a housing (13), to be secured to a container containing a sprayable liquid;
- a liquid ejecting member (3) connected to said housing and comprising a flexible member extending from an outer perimeter of said liquid ejecting member to an inner wall of the housing; wherein said liquid ejecting member is to be contacted with a liquid providing member (5) for providing liquid to said liquid ejecting member; and
- a vibrating actuator (2); said vibrating actuator is connected to said liquid ejecting member (3) for actuating said liquid ejecting member so as to generate a spray of liquid;
**characterized in that** a flexible connection between said housing and said liquid ejecting member is provided and that the flexible member (6) is arranged so as to provide a stable working contact between said liquid ejecting member (3) and said liquid providing member (5).

2. Liquid spray-head according to claim 1, **characterized in that** the flexible member (6) is annulus-shaped, extending around the liquid ejecting member (3).

3. Liquid spray-head according to claim 1 or 2, **characterized in that** the flexible member (6) is provided with a radial cross-section extending from an outer perimeter of said liquid ejecting member (3) to an inner wall of the housing for providing a flexible connection between said housing and said liquid ejecting member (3).

4. Liquid spray-head according to claim 3, **characterized in that** the flexible connection allows a certain amplitude of lateral movement along a line extending from the perimeter of the liquid ejecting member to the inner wall of said housing (13) so as to freely suspend the liquid ejecting member (3).

5. Liquid spray-head according to any of the preceding claims,
**characterized in that** the flexible member is provided with at least one undulation (61) between a central opening and its periphery.

6. Liquid spray-head according to any of the preceding claims,
**characterized in that** the flexible member (6) is cone-shaped, the cone extending in a direction normal to an ejection side of the liquid ejecting member.

7. Liquid spray-head, according to any of the preceding claims,
**characterized in that** the flexible member (6) comprises inwardly extending surface areas contacting opposite surfaces of said liquid ejecting member so as to enclose said liquid ejecting member (3).

8. Liquid spray-head according to any of the preceding claims,
**characterized in that** the flexible member (6) is provided with a inner perimeter that is matingly connected to a rim of the liquid ejecting member (3).

9. Liquid spray-head according to any of the preceding claims,
**characterized in that** the flexible member (6) is provided with an inner perimeter comprising a plurality of through-holes; the spray-head further comprising an enclosing member comprising protrusions entering said through-holes, for enclosing said liquid ejecting member (3) by said enclosing member and said flexible member.

10. Liquid spray-head according to any of the preceding claims,
**characterized in that** the inner perimeter comprises a notch (64) for passage of connecting wires of the vibrating actuator.

11. Liquid spray-head according to any of the preceding claims,
**characterized in that** the flexible member (6) is provided with a perimeter that is sealingly connectable to a rim of an opening of a liquid containing container (7).

12. Liquid spray-head according to any of the preceding claims,
**characterized in that** the flexible member (6) is provided with a reinforced perimeter.

13. Liquid spray-head according to any of the preceding claims, comprising a liquid providing member (5), the liquid spray-head further comprising an annular fixing member, for fixing said liquid providing member relative to the liquid ejecting member (3).

14. Liquid spray-head according to claim 13, **characterized in that** said liquid providing member comprises a chamfered surface (145) to be contacted with the liquid ejecting member.

15. Liquid spray-head according to any of the preceding claims,
**characterized in that** the flexible member is moulded from an elastomeric material from a group comprising silicones, fluorinated elastomers, fluorosilicones, nitrile-butadiene (NBR), hydrogenated nitrile-butadiene (HNBR), thermoplastic polyester-elastomer, neoprene (cloroprene), terpolimer of etylpropilene (EPDM) and EPDM peroxide.

16. Liquid spray-head according to claim 15, **characterized in that** the elastomeric material is the fluorinate elastomer FKM 60.

17. Liquid spray-head according to any of the preceding claims,
**characterized in that** the flexible member is monolithically formed to the housing.

18. Apparatus for spraying a liquid, comprising a liquid spray-head according to any of the preceding claims and a container for comprising liquid, to be secured to said liquid spray-head.

19. An apparatus according to claim 18, further comprising a liquid providing member and an annular fixing member, for fixing said liquid providing member.

20. An apparatus according to claim 19, wherein the fixing member (7A) comprises an outer surface providing a sealing contact between the periphery of a flexible member of a spray-head and an inner surface fixedly contacting said liquid providing member.

21. An apparatus according to claims 19 or 20, wherein the liquid providing member (5) has a chamfered surface (145) to be contacted with the liquid ejecting member.

22. Apparatus according to claim 21, **characterized in that** the apparatus comprises an electrical control circuit for controlling the liquid spray head, the control circuit comprising a timer circuit and/or an intensity switch.

23. Apparatus according to claim 21 or 22, **characterized in that** the apparatus comprises a fan (16) and a conduit for ducting fanned air over the spray-head.

24. Apparatus according to any of the claims 21-23, **characterized in that** the apparatus delivers fragrances.

25. Apparatus according to claims 24, **characterized in that** the fan comprises a rotorwheel having an axis of rotation parallel to an ejecting direction of the spray-head, wherein the conduit deflects fanned air in a direction transverse to said ejection direction.

26. Apparatus according to claim 24 or 25, wherein the control circuit is arranged to control the spray-head in at least two different operation modes previously programmed and stored in a memory associated to said control circuit, wherein in a first operation mode a first predetermined dose of liquid is sprayed during a first predetermined period of time for detection of crawling insects, and wherein in a second operation mode a second predetermined dose of liquid is sprayed during a second predetermined period of time, separated by a predetermined period from said first predetermined period of time, for effective extermination of crawling insects.

27. Apparatus according to claim 26, wherein said operation modes are selectable by a selector switch (32).

## Patentansprüche

1. Flüssigkeitszerstäuberkopf mit:
- einem Gehäuse (13) zur Befestigung an einem Behälter, der eine sprühbare Flüssigkeit enthält;
- einem Flüssigkeitsausstoßteil (3), das mit dem Gehäuse verbunden ist und ein flexibles Teil aufweist, welches von einem Außenumfangsbereich des Flüssigkeitsausstoßteils zu einer Innenwand des Gehäuses absteht, wobei das Flüssigkeitsausstoßteil mit einem Flüssigkeitszuführteil (5) zum Zuführen von Flüssigkeit zu dem Flüssigkeitsausstoßteil kontaktierbar ist; und
- einem Vibrationsbetätigungsteil (2); wobei das Vibrationsbetätigungsteil mit dem Flüssigkeitsausstoßteil (3) verbunden ist, um das Flüssigkeitsausstoßteil zu betätigen und **dadurch** einen Flüssigkeitssprühnebel zu erzeugen;
**dadurch gekennzeichnet, dass** eine flexible Verbindung zwischen dem Gehäuse und dem Flüssigkeitsausstoßteil vorgesehen ist und dass das flexible Teil (6) derart angeordnet ist, dass es einen stabilen Arbeitskontakt zwischen dem Flüssigkeitsausstoßteil (3) und dem Flüssigkeitszuführteil (5) bildet.

2. Flüssigkeitszerstäuberkopf nach Anspruch 1, **dadurch gekennzeichnet, dass** das flexible Teil (6) ringförmig ist, wobei es um das Flüssigkeitsausstoßteil (3) herum verläuft.

3. Flüssigkeitszerstäuberkopf nach Anspruch 1 oder 2, **dadurch gekennzeichnet, dass** das flexible Teil (6) einen radialen Querschnitt hat, der sich von einem Außenumfangsbereich des Flüssigkeitsausstoßteils (3) zu einer Innenwand des Gehäuses erstreckt, um eine flexible Verbindung zwischen dem Gehäuse und dem Flüssigkeitsausstoßteil (3) zu schaffen.

4. Flüssigkeitszerstäuberkopf nach Anspruch 3, **dadurch gekennzeichnet, dass** die flexible Verbindung eine gewisse Lateralbewegungsamplitude entlang einer vom Umfang des Flüssigkeitsausstoßteils zu einer Innenwand des Gehäuses (13) verlaufenden Linie zulässt, um eine freie Aufhängung des Flüssigkeitsausstoßteils (3) zu bewirken.

5. Flüssigkeitszerstäuberkopf nach einem der vorhergehenden Ansprüche,
**dadurch gekennzeichnet, dass** das flexible Teil mit mindestens einem Wellenbereich (61) zwischen einer zentralen Öffnung und seinem Umfangsbereich versehen ist.

6. Flüssigkeitszerstäuberkopf nach einem der vorhergehenden Ansprüche,
**dadurch gekennzeichnet, dass** das flexible Teil (6) konusförmig ist, wobei sich der Konus in einer normal zu einer Ausstoßseite des Flüssigkeitsausstoßteils verlaufenden Richtung erstreckt.

7. Flüssigkeitszerstäuberkopf nach einem der vorhergehenden Ansprüche,
**dadurch gekennzeichnet, dass** das flexible Teil (6) nach innen abstehende Flächenbereiche aufweist, die gegenüberliegende Flächen des Flüssigkeitsausstoßteils derart kontaktieren, dass sie das Flüssigkeitsausstoßteil (3) umschließen.

8. Flüssigkeitszerstäuberkopf nach einem der vorhergehenden Ansprüche,
**dadurch gekennzeichnet, dass** das flexible Teil (6) einen Innenumfangsbereich aufweist, der passend mit einem Rand des Flüssigkeitsausstoßteils (3) verbunden ist.

9. Flüssigkeitszerstäuberkopf nach einem der vorhergehenden Ansprüche,
**dadurch gekennzeichnet, dass** das flexible Teil (6) einen Innenumfangsbereich mit mehreren Durchgangsöffnungen aufweist, wobei der Sprühkopf ferner ein Umschließungsteil aufweist, das mit in die Durchgangsöffnungen ragenden Vorsprüngen versehen ist, derart, dass das Flüssigkeitsausstoßteil (3) von dem Umschließungsteil und dem flexiblen Teil umschlossen ist.

10. Flüssigkeitszerstäuberkopf nach einem der vorhergehenden Ansprüche,
**dadurch gekennzeichnet, dass** der Innenumfangsbereich eine Nut (64) für den Durchtritt von Verbindungsdrähten des Vibrationsbetätigungsteils aufweist.

11. Flüssigkeitszerstäuberkopf nach einem der vorhergehenden Ansprüche,
**dadurch gekennzeichnet, dass** das flexible Teil (6) einen Umfangsbereich aufweist, der dichtend mit einem Rand einer Öffnung eines Flüssigkeitsaufnahmebehälters (7) verbindbar ist.

12. Flüssigkeitszerstäuberkopf nach einem der vorhergehenden Ansprüche,
**dadurch gekennzeichnet, dass** das flexible Teil (6) mit einem verstärkten Umfangsbereich versehen ist.

13. Flüssigkeitszerstäuberkopf nach einem der vorhergehenden Ansprüche, mit einem Flüssigkeitszuführteil (5), wobei der Flüssigkeitszerstäuberkopf ferner ein ringförmiges Fixierteil zum Fixieren des Flüssigkeitszuführteils relativ zu dem Flüssigkeitsausstoßteil (3) aufweist.

14. Flüssigkeitszerstäuberkopf nach Anspruch 13, **dadurch gekennzeichnet, dass** das Flüssigkeitszuführteil eine vom Flüssigkeitsausstoßteil zu kontaktierende abgeschrägte Fläche (145) aufweist.

15. Flüssigkeitszerstäuberkopf nach einem der vorhergehenden Ansprüche,
**dadurch gekennzeichnet, dass** das flexible Teil aus einem Elastomermaterial geformt ist, das gewählt ist aus der Gruppe, zu der Silicone, fluorierte Elastomere, Fluorsilicone, Nitrilbutadien (NBR), hydriertes Nitrilbutadien (HNBR), thermoplastisches Polyesterelastomer, Neopren (Chloropren), Terpolymer von Ethylpropylen (EPDM) und EPDM-Peroxid zählen.

16. Flüssigkeitszerstäuberkopf nach Anspruch 15, **dadurch gekennzeichnet, dass** das Elastomermaterial das Fluorinat-Elastomer FKM 60 ist.

17. Flüssigkeitszerstäuberkopf nach einem der vorhergehenden Ansprüche,
**dadurch gekennzeichnet, dass** das flexible Teil dem Gehäuse monolitisch angeformt ist.

18. Vorrichtung zum Zerstäuben einer Flüssigkeit, mit einem Flüssigkeitszerstäuberkopf nach einem der vorhergehenden Ansprüche und einem am Flüssigkeitszerstäuberkopf befestigbaren Behälter zur Aufnahme von Flüssigkeit.

19. Vorrichtung nach Anspruch 18, ferner mit einem Flüssigkeitszuführteil und einem ringförmigen Fixierteil zum Fixieren des Flüssigkeitszuführteils.

20. Vorrichtung nach Anspruch 19, bei der das Fixierteil (7A) eine Außenfläche aufweist, die einen Dichtkontakt zwischen dem Umfangsbereich eines flexiblen Teils eines Zerstäuberkopfs und einer das Flüssigkeitszuführteil fest kontaktierenden Innenfläche bildet.

21. Vorrichtung nach Anspruch 19 oder 20, bei der das Flüssigkeitszuführteil (5) eine vom Flüssigkeitsausstoßteil zu kontaktierende abgeschrägte Fläche (145) aufweist.

22. Vorrichtung nach Anspruch 21, **dadurch gekennzeichnet, dass** die Vorrichtung eine elektrische Steuerschaltung zum Steuern des Flüssigkeitszerstäuberkopfs aufweist, wobei die Steuerschaltung eine Zeitgeberschaltung und/oder einen Intensitätsschalter aufweist.

23. Vorrichtung nach Anspruch 21 oder 22, **dadurch gekennzeichnet, dass** die Vorrichtung einen Ventilator (16) und eine Leitung zum Leiten von Ventilationsluft über den Zerstäuberkopf aufweist.

24. Vorrichtung nach einem der Ansprüche 21 - 23, **dadurch gekennzeichnet, dass** die Vorrichtung Düfte zuführt.

25. Vorrichtung nach Anspruch 24, **dadurch gekennzeichnet, dass** der Ventilator ein Rotorrad mit einer parallel zur Ausstoßrichtung des Sprühkopfs verlaufenden Drehachse aufweist, wobei die Leitung Ventilationsluft in einer quer zur Ausstoßrichtung verlaufenden Richtung umlenkt.

26. Vorrichtung nach Anspruch 24 oder 25, **dadurch gekennzeichnet, dass** die Steuerschaltung derart ausgebildet ist, dass sie den Zerstäuberkopf in mindestens zwei verschiedenen Betriebsarten steuert, die vorprogrammiert und in einem mit der Steuerschaltung verbundenen Speicher gespeichert sind, wobei in einer ersten Betriebsart zwecks Detektierens kriechender Insekten eine erste vorbestimmte Flüssigkeitsdosis während einer ersten vorbestimmten Zeitperiode gesprüht wird, und wobei in einer zweiten Betriebsart zwecks wirksamen Abtötens kriechender Insekten eine zweite vorbestimmte Flüssigkeitsdosis während einer zweiten vorbestimmten Zeitperiode gesprüht wird, die von der ersten vorbestimmten Zeitperiode um eine vorbestimmte Periode getrennt ist.

27. Vorrichtung nach Anspruch 26, bei der die Betriebsarten durch einen Wählschalter (32) wählbar sind.

## Revendications

1. Tête de vaporisation pour liquide, comprenant :
- un logement (13) à fixer sur un récipient contenant un liquide vaporisable ;
- un élément d'expulsion de liquide (3) raccordé au dit logement et comprenant un élément flexible s'étendant à partir d'un périmètre extérieur dudit élément d'expulsion de liquide jusqu'à une paroi intérieure du logement ; où ledit élément d'expulsion de liquide doit être mis en contact avec un élément de fourniture de liquide (5) destiné à fournir du liquide au dit élément d'expulsion de liquide ; et
- un actionneur vibrant (2), lequel actionneur vibrant est raccordé au dit élément d'expulsion de liquide (3) pour actionner ledit élément d'expulsion de liquide de manière à produire un brouillard de liquide ;
**caractérisée en ce qu'**un raccord flexible est prévu entre ledit logement et ledit élément d'expulsion de liquide et **en ce que** l'élément flexible (6) est agencé de manière à réaliser un contact fonctionnel stable entre ledit élément d'expulsion de liquide (3) et ledit élément de fourniture de liquide (5).

2. Tête de vaporisation pour liquide selon la revendication 1, **caractérisée en ce que** l'élément flexible (6) présente une forme annulaire s'étendant autour de l'élément d'expulsion de liquide (3).

3. Tête de vaporisation pour liquide selon l'une des revendications 1 et 2, **caractérisée en ce que** l'élément flexible (6) présente une section transversale radiale s'étendant à partir d'un périmètre extérieur dudit élément d'expulsion de liquide (3) jusqu'à une paroi intérieure du logement afin de fournir un raccord flexible entre ledit logement et ledit élément d'expulsion de liquide (3).

4. Tête de vaporisation pour liquide selon la revendication 3, **caractérisée en ce que** le raccord flexible permet une certaine amplitude de mouvement latéral le long d'une ligne s'étendant à partir du périmètre de l'élément d'expulsion de liquide jusqu'à la paroi intérieure dudit logement (13) de manière à suspendre librement l'élément d'expulsion de liquide (3).

5. Tête de vaporisation pour liquide selon l'une quelconque des revendications précédentes, **caractérisée en ce que** l'élément flexible comporte au moins une ondulation (61) entre une ouverture centrale et sa périphérie.

6. Tête de vaporisation pour liquide selon l'une quelconque des revendications précédentes, **caractérisée en ce que** l'élément flexible (6) présente une forme conique, le cône s'étendant dans une direction normale vers un côté d'expulsion de l'élément d'expulsion de liquide.

7. Tête de vaporisation pour liquide selon l'une quelconque des revendications précédentes, **caractérisée en ce que** l'élément flexible (6) comprend des zones de surface s'étendant vers l'intérieur faisant contact avec des surfaces opposées dudit élément d'expulsion de liquide de manière à enfermer ledit élément d'expulsion de liquide (3).

8. Tête de vaporisation pour liquide selon l'une quelconque des revendications précédentes, **caractérisée en ce que** l'élément flexible (6) présente un périmètre intérieur qui est raccordé de façon étroite à une couronne de l'élément d'expulsion de liquide (3).

9. Tête de vaporisation pour liquide selon l'une quelconque des revendications précédentes, **caractérisée en ce que** l'élément flexible (6) comporte un périmètre intérieur comprenant une pluralité de trous passants, la tête de vaporisation comprenant en outre un élément d'enceinte comprenant des saillies pénétrant dans lesdits trous passants, aux fins d'enfermer ledit élément d'expulsion de liquide (3) par ledit élément d'enceinte et ledit élément flexible.

10. Tête de vaporisation pour liquide selon l'une quelconque des revendications précédentes, **caractérisée en ce que** le périmètre intérieur comprend une encoche (64) de passage de câbles de raccordement de l'actionneur vibrant.

11. Tête de vaporisation pour liquide selon l'une quelconque des revendications précédentes, **caractérisée en ce que** l'élément flexible (6) présente un périmètre qui peut être raccordé en faisant étanchéité à une couronne d'une ouverture d'un récipient contenant du liquide (7).

12. Tête de vaporisation pour liquide selon l'une quelconque des revendications précédentes, **caractérisée en ce que** l'élément flexible (6) comporte un périmètre renforcé.

13. Tête de vaporisation pour liquide selon l'une quelconque des revendications précédentes, comprenant un élément de fourniture de liquide (5), la tête de vaporisation pour liquide comprenant en outre un élément de fixation annulaire destiné à fixer ledit élément de fourniture de liquide par rapport à l'élément d'expulsion de liquide (3).

14. Tête de vaporisation pour liquide selon la revendication 13, **caractérisée en ce que** ledit élément de fourniture de liquide comprend une surface chanfreinée (145) pour être mis en contact avec l'élément d'expulsion de liquide.

15. Tête de vaporisation pour liquide selon l'une quelconque des revendications précédentes, **caractérisée en ce que** l'élément flexible est moulé à partir d'un matériau élastomère choisi parmi un groupe comprenant les silicones, les élastomères fluorés, les fluorosilicones, le nitrile butadiène (NBR), le nitrile butadiène hydrogéné (HNBR), l'élastomère polyester thermoplastique, le néoprène (chloroprène), le terpolymère éthylène-propylène (EPDM) et le peroxyde d'EPDM.

16. Tête de vaporisation pour liquide selon la revendication 15, **caractérisée en ce que** le matériau élastomère est l'élastomère fluoré FKM 60.

17. Tête de vaporisation pour liquide selon l'une quelconque des revendications précédentes, **caractérisée en ce que** l'élément flexible est formé de façon monolithique avec le logement.

18. Appareil pour vaporiser un liquide, comprenant une tête de vaporisation pour liquide selon l'une quelconque des revendications précédentes et un récipient pour contenir le liquide à fixer à ladite tête de vaporisation pour liquide.

19. Appareil selon la revendication 18, comprenant en outre un élément de fourniture de liquide et un élément de fixation annulaire pour fixer ledit élément de fourniture de liquide.

20. Appareil selon la revendication 19, dans lequel l'élément de fixation (7A) comprend une surface extérieure réalisant un contact d'étanchéité entre la périphérie d'un élément flexible d'une tête de vaporisation et une surface intérieure faisant contact de façon fixe avec ledit élément de fourniture de liquide.

21. Appareil selon l'une des revendications 19 et 20, dans lequel l'élément de fourniture de liquide (5) présente une surface chanfreinée (145) pour être mis en contact avec l'élément d'expulsion de liquide.

22. Appareil selon la revendication 21, **caractérisé en ce que** l'appareil comprend un circuit de commande électrique pour commander la tête de vaporisation de liquide, le circuit de commande comprenant un circuit de minuterie et/ou un commutateur d'intensité.

23. Appareil selon l'une des revendications 21 et 22, **caractérisé en ce que** l'appareil comprend un ventilateur (16) et un conduit pour amener l'air soufflé au-dessus de la tête de vaporisation.

24. Appareil selon l'une quelconque des revendications 21 à 23, **caractérisé en ce que** l'appareil émet des parfums.

25. Appareil selon la revendication 24, **caractérisé en ce que** le ventilateur comprend une roue mobile ayant un axe de rotation parallèle à une direction d'expulsion de la tête de vaporisation, où le conduit dévie l'air soufflé dans une direction transversale à ladite direction d'expulsion.

26. Appareil selon l'une des revendications 24 et 25, dans lequel le circuit de commande est conçu pour commander la tête de vaporisation dans au moins deux modes de fonctionnement différents programmés préalablement et enregistrés dans une mémoire associée au dit circuit de commande, où, dans un premier mode de fonctionnement, une première dose prédéterminée de liquide est vaporisée pendant une première durée prédéterminée pour la détection d'insectes rampants, et où, dans un deuxième mode de fonctionnement, une deuxième dose prédéterminée de liquide est vaporisée pendant une deuxième durée prédéterminée, séparée de ladite première durée prédéterminée par une durée prédéterminée, pour l'extermination effective d'insectes rampants.

27. Appareil selon la revendication 26, dans lequel lesdits modes de fonctionnement peuvent être sélectionnés par un commutateur de sélection (32).
